Europäisches Patentamt

European Patent Office

Office européen des brevets

(11) Publication number: **0 092 314**

**B1**

(12) **EUROPEAN PATENT SPECIFICATION**

(45) Date of publication of patent specification: **02.07.86**

(21) Application number: **83301358.4**

(22) Date of filing: **11.03.83**

(51) Int. Cl.⁴: **A 61 B 17/58**

(54) **Orthopaedic apparatus.**

(30) Priority: **18.03.82 GB 8207900**

(43) Date of publication of application:
**26.10.83 Bulletin 83/43**

(45) Publication of the grant of the patent:
**02.07.86 Bulletin 86/27**

(84) Designated Contracting States:
**CH DE FR GB LI**

(56) References cited:
**FR-A-2 473 877**
**GB-A- 138 783**
**US-A-2 432 695**

**ENGINEERING IN MEDICINE, vol. 9, no. 2, April
1980, pages 84-86, MEP Ltd., London, G.B. A.J.
BANKS et al.: "The Salford technique for the
treatment of complicated tibial fractures"**

(73) Proprietor: **OEC EUROPE LIMITED
134 Brompton Road
London, S.W.3. (GB)**

(72) Inventor: **Denham, Robin Arthur
The Manor House
Droxford Hampshire (GB)**

(74) Representative: **Burrows, Anthony Gregory
et al
Haseltine Lake & Co. Hazlitt House 28
Southampton Buildings Chancery Lane
London, WC2 1AT (GB)**

Courier Press, Leamington Spa, England.

## Description

This invention relates to orthopaedic apparatus.

Known apparatus for use in external fixation of a bone (and disclosed in, for example, United States Patent Specification 2,432,695) includes two carriages in the form of elongate metal blocks for attaching to bone at respective opposite sides of a fracture, for example of the tibia, by way of pins or screws surrounded by bone cement. Each carriage is formed with an internal keyed bore extending therealong, and through these two bores can be slid a straight rigid steel rod formed throughout its length with a screw-thread for co-operating with four nuts, one at each end of each carriage. The rod is also formed throughout its length with a keyway for receiving the keys of the two carriages. By screwing the nuts inwardly towards each other, the carriages can be displaced towards each other along the rod, so to put the fracture under compression.

After the fixation operation, it may be found that the bone parts at respective opposite sides of the fracture are angularly misaligned, for example because one of the carriages is not correctly orientated on its bone part. In order to correct this misalignment, it is likely to be necessary to detach the one carriage from its bone part and to re-attach it with the correct orientation.

In an article "The Salford technique for the treatment of complicated tibial fractures" in the journal "Engineering in Medicine", Volume 9, No. 2, April 1980, pages 84—86, published by MEP Limited, London, United Kingdom, there is disclosed an external fixation apparatus comprised of two threaded rods fixed to a ball and a locking pad, respectively, received in a housing formed internally with a spherical seating face on which the ball can turn. By means of socket head cap screws screwed through the housing wall into conical recesses in a cylindrical external peripheral surface of the locking pad closely encircled by a cylindrical internal peripheral surface of the housing, the locking pad is pressed against the ball in order to clamp the ball between the seating face and the pad to prevent turning of the ball relative to the housing and the pad. However, if the screws are not fully tightened, or loosen postoperatively, the ball can turn relative to the housing and thus the bone parts turn out of alignment.

According to one aspect of the present invention, as defined in independent claim 1, there is provided an orthopaedic apparatus for use in external fixation of a bone, comprising first and second members for attaching to bone at respective opposite sides of a fracture by means of fastening elements, and a straight rigid elongate means releasably mountable on the first and second members for maintaining the first and second members fixed relative to each other, characterized in that the apparatus includes another elongate means for replacing said rigid elongate means to maintain the first and second members fixed relative to each other and comprised of first and second rigid parts releasably mountable on the first and second members, respectively, and interconnecting means arranged so to interconnect the first and second parts as to enable adjustment of the relative angular position of those parts and fixing of the adjusted angular position, and in that said interconnecting means comprises a first plurality of holes formed through the first part and distributed around an axis of relative turning of the first and second parts at substantially a constant radius from said axis, a second plurality of holes formed in the second part and distributed around said axis at substantially said constant radius but with a pitch different from that of the first plurality, and a locating member insertable through a selected one of said first plurality into a selected one of said second plurality.

It is thereby possible for a surgeon to replace the rigid elongate means by the other elongate means and ensure correct bone alignment without the need to detach one of the first and second members from the bone. Moreover, since the apparatus does not rely upon friction between the first and second parts to prevent relative turning thereof, the correct bone alignment is reliably maintained thereafter.

According to another aspect of the present invention, as defined in independent claim 6, there is provided orthopaedic apparatus for use in obtaining and maintaining bone alignments, comprising first and second elements turnable relative to each other about an axis, characterized in that a first plurality of holes is formed through the first element and distributed around said axis at substantially a constant radius therefrom, a second plurality of holes is formed in the second element and distributed around said axis at substantially said constant radius but with a pitch different from that of the first plurality, and a locating member is insertable through a selected one of said first plurality into a selected one of said second plurality.

This orthopaedic apparatus is usable in various applications where bone alignments should be relatively accurately obtained and reliably maintained thereafter.

In order that the invention may be clearly understood and readily carried into effect, reference will now be made, by way of example, to the accompanying drawings, in which:—

Figure 1 shows a sectional side elevation of a device of an apparatus for use in external fixation,

Figure 2 shows a top plan view of the device, and

Figure 3 shows a fragmentary exploded view of two assemblies and a locating pin of the device, the left-hand assembly being shown in underneath plan, the right-hand assembly in top plan and the pin in side elevation.

The apparatus for use in external fixation includes two carriages in the form of elongate metal blocks (not shown) for attaching to bone at respective opposite sides of a fracture, for example of the tibia, by way of pins or screws surrounded by cement. Each metal block is formed with an internal keyed bore extending therealong, and through these bores can be slid a straight rigid steel rod formed throughout its length with a screw-thread for co-operating with four nuts, one at each end of each block. The rod is also formed throughout its length with a keyway for receiving the keys of the blocks. By screwing the nuts inwardly towards each other, the blocks can be displaced towards each other along the rod, so to put the fracture under compression.

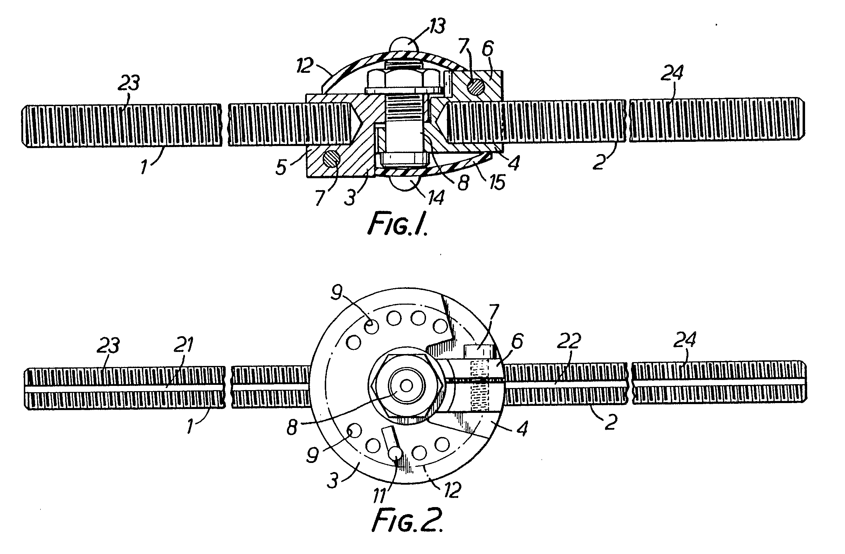

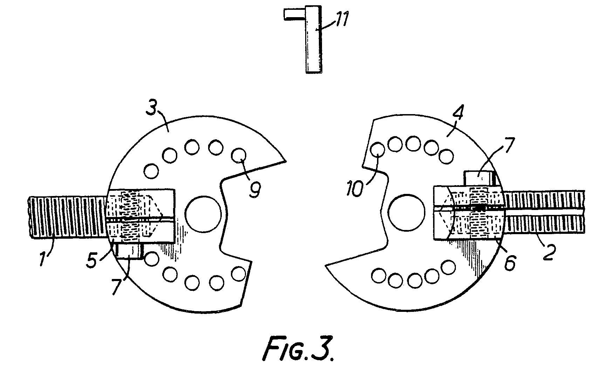

After the fixation operation, it may be found that the bone parts at respective sides of the fracture are angularly misaligned, for example because one of the blocks is not correctly orientated on its bone part. In order to enable this to be corrected, the new device shown in Figures 1 to 3 is provided. It is designed to replace the straight rod, without it being necessary for either of the blocks to be detached from the bone. It comprises two screw-threaded rods 1 and 2, which are formed with respective longitudinal keyways 21 and 22 and are identical to each other, except that their screwthreads 23 and 24 are of opposite hand. The device also includes two part-circular aluminium or steel elements 3 and 4 which incorporate respective U-clamps 5 and 6 which, by means of bolts 7, enable the elements 3 and 4 to be clamped to the respective inner ends of the rods 1 and 2. The elements 3 and 4 are connected together by a nut-and-bolt device 8 having its axis perpendicular to the rods 1 and 2. The elements 3 and 4 are formed with through bores 9 and 10 all parallel to the axis of the device 8. The bores 9 and 10 are arranged distributed about the axis of the device 8 at a constant radius from that axis. Moreover, the bores 9 or 10 of each element 3 or 4 are arranged in two sets separated by the U-clamp 5 or 6. The bores 9 in each set are equiangularly spaced around the axis of the device 8, as are the bores 10 in each set, except that the angle of spacing of the bores 9 is greater than the angle of the spacing of the bores 10. The arrangement, particularly the angular spacing, is such that, for every few degrees of rotation of the elements 3 and 4 relative to each other around the axis of the device 8 throughout a range of about 20° to each side of the 180° position of the rods 1 and 2, at least one of the bores 9 comes into exact alignment with at least one of the bores 10. With the desired bores 9 and 10 in exact alignment, a locating pin 11 can be inserted into those bores 9 and 10 to lock the elements 3 and 4 in the adjusted position. The pin is prevented from working out of the bores 9 and 10 by means of a plastics cap 12 held in position at one end of the bolt of the device 8 by a screw 13 extending axially through the bolt of the device 8. At the other end of that bolt, a nut 14 screwed onto the screw 13 holds another cap 15 in position. In Figure 2, the plastics cap 12 is shown in outline only.

Following removal of the straight rod from the elongate blocks, the rods 1 and 2 are inserted through the blocks, four nuts being screwed onto the rods 1 and 2, one at each end of each block so as to leave the blocks loose between them. The rods 1 and 2 are inserted into the elements 3 and 4, with the items 3, 4 and 8 being in the assembled condition shown in Figures 1 and 2, the device 8 being loose enough to allow the elements 3 and 4 to turn relative to each other. With the clamps 5 and 6 untightened, these assembled items are turned as a unit about the axes of the rods 1 and 2, to bring the plane of angular adjustment of the elements 3 and 4 into a position parallel to the plane of correction needed between the two bone parts. Thereupon, with the rods 1 and 2 inserted deeply into the elements 3 and 4, the clamps 5 and 6 are tightened upon the rods 1 and 2, and the rods 1 and 2 are turned about the device 8 relative to each other to align the bone parts, the pin 11 being inserted into those two bores 9 and 10 giving the best degree of alignment of the bone parts. Thereupon, the device 8 is tightened, the four nuts are tightened against the two blocks, and the caps 12 and 14 are placed in position and retained by the screw 13 and the nut 14.

Among the advantages of the apparatus hereinbefore described are that it and thus the fracture can be adjusted as regards angular position without removal of the elongate blocks; a relatively high degree of correction of the angular position can be reliably obtained, and, following adjustment, the apparatus is robust and remains in the adjusted condition.

**Claims**

1. An orthopaedic apparatus for use in external fixation of a bone, comprising first and second members for attaching to bone at respective opposite sides of a fracture by means of fastening elements, and a straight rigid elongate means releasably mountable on the first and second members for maintaining the first and second members fixed relative to each other, characterized in that the apparatus includes another elongate means (1—11) for replacing said rigid elongate means to maintain the first and second members fixed relative to each other and comprised of first and second rigid parts (1—7) releasably mountable on the first and second members, respectively, and interconnecting means (8—11) arranged so to interconnect the first and second parts (1—7) as to enable adjustment of the relative angular position of those parts (1—7) and fixing of the adjusted angular position, and in that said interconnecting means (8—11) comprises a first plurality of holes (9) formed through the first part (1, 3, 5, 7) and distributed around an axis of relative turning of the first and second parts (1—7) at substantially a constant radius from said axis, a second plurality of holes (10) formed in the second part (2, 4, 6, 7) and distributed around said axis at substantially said constant radius but with a pitch different from that of the first plurality (9), and a locating member (11) insertable through a selected one of said first plurality (9) into a selected one of said second plurality (10).

2. Apparatus according to claim 1, wherein said interconnecting means includes readily removable hinge pin means (8) defining said axis.

3. Apparatus according to claim 2, wherein said hinge pin means (8) serves also to clamp releasably the first rigid part (1, 3, 5, 7) to the second rigid part (2, 4, 6, 7).

4. Apparatus according to any preceding claim, and further comprising protective cap means (12, 15) arranged to cover the first and second pluralities of holes (9, 10) and the locating member (11) and to retain said locating member (11) in the selected holes (9, 10) and fastening means (13, 14) arranged releasably to fasten said protective cap means (12, 15) to said other elongate means (1—11).

5. Apparatus according to any preceding claim, wherein each of the first and second rigid parts (1—7) comprises a turnable hinge member (3, 4), an elongate member (12), and clamping means (5, 6, 7) arranged releasably to clamp said hinge member (3, 4) to one end of said elongate member (1, 2).

6. Orthopaedic apparatus for use in obtaining and maintaining bone alignments, comprising first and second elements (3, 4) turnable relative to each other about an axis, characterized in that a first plurality of holes (9) is formed through the first element (3) and distributed around said axis at substantially a constant radius therefrom, a second plurality of holes (10) is formed in the second element (4) and distributed around said axis at substantially said constant radius but with a pitch different from that of the first plurality (9), and a locating member (11) is insertable through a selected one of said first plurality (9) into a selected one of said second plurality (10).

7. Apparatus according to claim 6, and further comprising protective cap means (12, 15) arranged to cover the first and second plurality of holes (9, 10) and the locating member (11) and to retain said locating member (11) in the selected holes (9, 10), and fastening means (13, 14) arranged releasably to fasten said protective cap means (12, 15) to at least one of said first and second elements (3, 4).

8. Apparatus according to claim 6 or 7, and further comprising readily removable hinge pin means (8) arranged pivotally to interconnect said first and second elements (3, 4) and defining said axis.

9. Apparatus according to claim 6 or 7, and further comprising releasable clamping means (8) arranged to clamp releasably said first element (3) to said second element (4).

## Revendications

1. Appareil orthopédique destiné à être utilisé pour la fixation externe d'un os, comprenant un premier et un deuxième élément destinés à être fixés sur l'os à des côtés opposés respectifs d'une fracture au moyen d'éléments de fixation, et un moyen allongé rigide rectiligne, pouvant se monter de manière amovible sur le premier et le deuxième élément, pour maintenir les premier et deuxième éléments fixes l'un par rapport à l'autre, appareil caractérisé en ce qu'il comporte un autre élément allongé (1—11) pour remplacer ledit élément allongé rigide de façon à maintenir le premier et le deuxième élément fixes l'un par rapport à l'autre, et composé d'un premier et d'un deuxième élément rigides (1—7) pouvant se monter de façon amovible sur le premier et le deuxième élément, respectivement, et un moyen d'interconnexion (8—11) disposé de façon à relier le premier et le deuxième élément (1—7) de façon à permettre le réglage de la position angulaire relative de ces éléments (1—7), et la fixation de la position angulaire réglée, et en ce que le moyen d'interconnexion (8—11) se compose d'une première pluralité de trous (9) formés à travers la première partie (1, 3, 5, 7) et répartis autour d'un axe de rotation relative du premier et du deuxième élément (1—7) à un rayon essentiellement constant par rapport au dit axe, une deuxième pluralité de trous (10) étant formée dans le deuxième élément (2, 4, 6, 7) et répartis autour dudit axe à un rayon essentiellement constant, mais d'un pas différent de celui de la première pluralité de trous (9), un élément de positionnement (11) pouvant être inséré à travers un trou choisi dans la première pluralité de trous (9) et dans un trou choisi de la deuxième pluralité de trous (10).

2. Appareil selon revendication 1, sur lequel le moyen d'interconnexion comporte un moyen d'articulation (8) facilement amovible définissant ledit axe.

3. Appareil selon revendication 2, sur lequel le moyen d'articulation (8) sert également à brider de manière amovible le premier élément rigide (1, 3, 5, 7) sur le second élément rigide (2, 4, 6, 7).

4. Appareil selon l'une quelconque des revendications précédentes, comportant en plus un moyen à capuchon de protection (12, 15) disposé de façon à recouvrir la première et la deuxième pluralité de trous (9, 10) et l'élément de positionnement (11) et à retenir ledit élément de positionnement (11) dans les trous choisis (9, 10) et des moyens de fixation (13, 14) disposés de façon amovible pour fixer ledit moyen à capuchon de protection (12, 15) sur ledit moyen allongé (1—11).

5. Appareil selon l'une quelconque des revendications précédentes, sur lequel chacun des premier et deuxième éléments rigides (1—7) comportent un élément d'articulation rotatif (3, 4), un élément allongé (12), et des moyens de bridage (5, 6, 7) disposé de façon amovible pour brider l'élément articulé (3, 4) sur une extrémité dudit élément allongé (1, 2).

6. Appareil orthopédique destiné à être utilisé pour obtenir et maintenir des alignement d'os, comprenant un premier et un deuxième élément (3, 4), rotatifs l'un par rapport à l'autre autour d'un axe, caractérisé en ce qu'une première pluralité de trous (9) sont formés à travers le premier élément 3 et répartis autour dudit axe à un rayon essentiellement constant, une deuxième pluralité de trous (10) sont formés sur le second élément (4) et répartis autour dudit axe à un rayon essentiellement constant, mais d'un pas différent de celui de la première pluralité de trous (9), un élément de positionnement (11) pouvant s'insérer à travers un trou sélectionné sur la première pluralité de trous (9) dans un trou sélectionné sur la deuxième pluralité de trous (10).

7. Appareil selon revendication 6, comportant en outre des moyens à capuchon de protection (12, 15) disposés de façon à couvrir la première et la deuxième pluralité de trous (5, 10) et l'élément de positionnement (11) et pour retenir ledit élément de positionnement (11) dans les trous choisis (9, 10), et un moyen de fixation (13, 14) disposé de façon amovible pour fixer les moyens à capuchon de protection (12, 15) sur au moins un desdits premier et deuxième éléments (3, 4).

8. Appareil selon revendication 6 ou 7, comprenant en outre un moyen d'articulation facilement amovible (8) disposé de façon à pivoter et à relier le premier et le deuxième élément (3, 4) et définissant ledit axe.

9. Appareil selon revendication 6 ou 7, comportant en outre un moyen de bridage pouvant être desserré (8), prévu pour brider de manière desserrable ledit premier élément (3) sur ledit deuxième élément (4).

**Patentansprüche**

1. Orthopädisches Gerät zur Verwendung bei der externen Fixierung eines Knochens, mit ersten und zweiten Gliedern zum Anbringen an dem Knochen an den jeweiligen sich gegenüberliegenden Seiten einer Fraktur mittels Befestigungselementen, und einem geradlinigen steifen und länglichen Mittel, das lösbar an erstem und zweitem Glied anbringbar ist, um erstes und zweites Glied relativ zueinander fixiert zu halten, dadurch gekennzeichnet, daß das Gerät ein weiteres längliches Mittel (1—11) zum Ersetzen des steifen länglichen Mittels aufweist, um erstes und zweites Glied relativ zueinander fixiert zu halten, das aus ersten und zweiten steifen Teilen (1—7) besteht, die lösbar an dem ersten bzw. zweiten Glied anbringbar sind, sowie Verbindungsmittel (8—11) enthält, die so angeordnet sind, daß sie erstes und zweites Teil (1—7) derart miteinander verbinden, daß eine Einstellung der relativen Winkellagen dieser Teile (1—7) und eine Fixierung der eingestellten Winkellage möglich ist, und

daß die Verbindungsmittel (8—11) mehrere erste Löcher (9) durch das erste Teil (1, 3, 5, 7), die um eine Achse der relativen Drehung von erstem und zweitem Teil (1—7) mit im wesentlichen konstantem Radius gegenüber der Achse verteilt ausgebildet sind, mehrere zweite Löcher (10) in dem zweiten Teil (2, 4, 6, 7), die um die Achse mit im wesentlichen konstantem Radius, jedoch mit einer Schrittweite, die von der der mehreren ersten Löcher (9) verschieden ist, verteilt ausgebildet sind, sowie ein Arretierteil (11) aufweisen, das durch ein ausgewähltes Loch der mehreren ersten Löcher (9) in ein ausgewähltes Loch der mehreren zweiten Löcher (10) einsetzbar ist.

2. Gerät nach Anspruch 1, bei dem die Verbindungsmittel einen schnell entfernbaren Gelenkstift (8) aufweisen, der die Achse definiert.

3. Gerät nach Anspruch 2, bei dem der Gelenkstift (8) außerdem dazu dient, das erste steife Teil (1, 3, 5, 7) an dem zweiten steifen Teil (2, 4, 6, 7) lösbar festzumachen.

4. Gerät nach einem der vorhergehenden Ansprüche, das desweiteren Schutzkappen (12, 15), die so angeordnet sind, daß sie die mehreren ersten und zweiten Löcher (9, 10) und das Arretierteil (11) abdecken und das Arretierteil (11) in den ausgewählten Löchern (9, 10) festhalten, sowie Befestigungsmittel (13, 14) enthält, die lösbar angeordnet sind, um die Schutzkappen (12, 15) an dem weiteren länglichen Mittel (1—11) zu befestigen.

5. Gerät nach einem der vorhergehenden Ansprüche, bei dem jedes von erstem und zweitem steifen Teil (1—7) ein drehbares Gelenkteil (3, 4), ein längliches Teil (1, 2) und Befestigungsmittel (5, 6, 7) enthält, das lösbar angeordnet ist, um das Gelenkteil (3, 4) an einem Ende des länglichen Teils (1, 2) festzuhalten.

6. Orthopädisches Gerät zur Verwendung zum Erreichen und Aufrechterhalten von Knochenausrichtungen, mit ersten und zweiten Elementen (3, 4), die relativ zueinander um eine Achse verdrehbar sind, dadurch gekennzeichnet, daß mehrere erste Löcher (9) durch das erste Element (3) ausgebildet sind und um die Achse mit im wesentlichen konstanten Radius gegenüber dieser verteilt sind, daß mehrere zweite Löcher (10) in dem zweiten Element (4) ausgebildet sind und um die Achse mit dem im wesentlichen konstanten Radius, jedoch mit einer Schrittweite, die von der der mehreren ersten Löcher verschieden ist, verteilt sind, und daß ein Arretierteil (11) durch ein ausgewähltes der mehreren ersten Löcher (9) in ein ausgewähltes der mehreren zweiten Löcher (10) einsteckbar ist.

7. Gerät nach Anspruch 6, das desweiteren Schutzkappen (12, 15), die so angeordnet sind, daß sie die mehreren ersten und zweiten Löcher (9, 10) und das Arretierteil (11) abdecken und das Arretierteil (11) in den ausgewählten Löchern (9, 10) festhalten, sowie Befestigungsmittel (13, 14) enthält, die lösbar angeordnet sind, um die Schutzkappen (12, 15) an zumindest einem von erstem und zweitem Element (3, 4) zu befestigen.

8. Gerät nach Anspruch 6 oder 7, das desweiteren einen schnell entfernbaren Gelenkstift (8) enthält, der drehbar angeordnet sit, um erstes und zweites Element (3, 4) miteinander zu verbinden und um die Achse zu definieren.

9. Gerät nach Anspruch 6 oder 7, das desweiteren ein lösbares Haltemittel (8) enthält, das so angeordnet ist, daß es das erste Element (3) an dem zweiten Element (4) lösbar festhält.

*Fig.1.*

*Fig.2.*

1

FiG.3.